# EUROPÄISCHE PATENTANMELDUNG

(11) **EP 4 850 711 A1**
(43) Veröffentlichungstag der Anmeldung: **09.12.2026**
(21) Anmeldenummer: 26175660.5
(22) Anmeldetag: 30.04.2026
(51) Int. Cl.: A61M 1/00, A61B 3/16, G01L 9/00, G01L 19/00

(54) **DRUCKSENSOREINRICHTUNG, OPHTHALMOLOGISCHE KASSETTE UND SYSTEM**

(30) Priorität: 03.06.2025 DE 102025121618
(71) Anmelder: Geuder AG, 69126 Heidelberg (DE)
(72) Erfinder: Stegmaier, Lukas, 69226 Nußloch (DE); Schlund, Christopher, 69168 Wiesloch (DE); Prisching, Thomas, 68782 Brühl (DE); Reuter, Wolfgang, 61231 Bad Nauheim (DE)
(74) Vertreter: Ullrich & Naumann PartG mbB

(57) **Zusammenfassung**

Die Erfindung betrifft Drucksensoreinrichtung (1), insbesondere für eine ophthalmologische Kassette, mit einem Drucksensoreinlass (9) und einem Drucksensorauslass (17), wobei die Drucksensoreinrichtung (1) zumindest einen Boden (16) und eine Wandung (6) aufweist, wobei die Drucksensoreinrichtung (1) mit einer Drucksensormembran (3) ausgebildet ist, wobei in der Drucksensoreinrichtung (1) ein Strömungsleitmaßnahme (10) angeordnet ist.

## Beschreibung

Die Erfindung betrifft eine Drucksensoreinrichtung für eine ophthalmologische Kassette mit einem Drucksensoreinlass und einem Drucksensorauslass, wobei die Drucksensoreinrichtung zumindest einen Boden und eine Wandung aufweist.

Die Erfindung betrifft außerdem eine ophthalmologische Kassette, insbesondere für ein Phakoemulsifikationssystem, umfassend ein Gehäuse, zumindest eine Irrigationsleitung und einen Anschlag für eine Rollenpumpe, wobei das Gehäuse einen Boden und eine Wandung aufweist, wobei sich die Irrigationsleitung von einem Einlass entlang des Anschlags bis zu einem Auslass erstreckt, wobei in dem Auslass eine Drucksensoreinrichtung angeordnet ist.

Die Erfindung betrifft weiter ein System aus einer ophthalmologischen Kassette und einer ophthalmologischen Konsole.

Ophthalmologische Kassette der in Rede stehenden Art sind seit Jahren aus der Praxis bekannt. Diese werden bei Augenoperationen zum Leiten von Fluiden in das Auge hinein (sogenannte Irrigation) und aus dem Auge des Patienten heraus (sogenannte Aspiration) genutzt. Die Kassette wird dabei in eine ophthalmologische Konsole eingesetzt, wobei die Kassette und die Konsole zusammen ein System bilden. Im Inneren der Kassette sind Fluidwege gebildet, die eine Irrigationsleitung und/oder eine Aspirationsleitung bilden. Dabei sind die Irrigationsleitung und/oder die Aspirationsleitung als flexible Schläuche ausgebildet, die entlang eines bogenförmigen Anschlags verlaufen. An dem bogenförmigen Anschlag kann eine Rollenpumpe angeordnet werden, die die Irrigations- und/oder Aspirationsleitung walken kann, um so Fluide durch die Irrigations- und/oder Aspirationsleitung zu fördern. Die Rollenpumpe selbst ist dabei in der Konsole angeordnet.

Um ein Zusammenbrechen des Kapselsacks des Auges während der Operation zu verhindern, ist es notwendig, den Augeninnendruck genau zu kontrollieren. Hierfür ist es bekannt geworden, eine Drucksensormembran in einer Drucksensoreinrichtung vorzusehen, die den Druck innerhalb der Irrigations- und/oder Aspirationsleitung messen kann.

Nachteilig an dieser Anordnung ist, dass durch die Rollenpumpe Pulsationen des Drucks in der Irrigations- und/oder Aspirationsleitung entstehen können, die sich negativ auf den Augeninnendruck auswirken können. Außerdem sind diese Druckpulsationen schwierig zu messen. Darüber hinaus sollen ophthalmologische Kassetten kompakt und bauraumeffizient sein.

Der vorliegenden Erfindung liegt daher die Aufgabe zugrunde, eine Drucksensoreinrichtung, eine ophthalmologische Kassette und ein ophthalmologisches System der eingangs genannten Art derart auszugestalten und weiterzubilden, dass auf einfache und bauraumarme Weise die Druckpulsationen verringert werden können.

Erfindungsgemäß wird die voranstehende Aufgabe durch die Merkmale des Anspruches 1 gelöst. Danach ist eine Drucksensoreinrichtung vorgesehen, für eine ophthalmologische Kassette, mit einem Drucksensoreinlass und einem Drucksensorauslass, wobei die Drucksensoreinrichtung zumindest einen Boden und eine Wandung aufweist.

Die Erfindung ist dadurch gekennzeichnet, dass die Drucksensoreinrichtung mit einer Drucksensormembran ausgebildet ist, wobei in der Drucksensoreinrichtung ein Strömungsleitelement angeordnet ist.

Darüber hinaus wird die voranstehende Aufgabe durch die Merkmale des Anspruchs 12 gelöst. Danach ist ein Ophthalmologische Kassette angegeben, insbesondere für ein Phakoemulsifikationssystem, umfassend ein Gehäuse, eine Irrigationsleitung und einen Anschlag für eine Rollenpumpe, wobei sich die Irrigationsleitung von einem Einlass entlang des Anschlags bis zu einem Auslass erstreckt, wobei in dem Auslass eine Drucksensoreinrichtung gemäß einem der Ansprüche 1 bis 11 angeordnet ist.

Darüber hinaus wird die voranstehende Aufgabe durch die Merkmale des Anspruchs 14 gelöst. Danach ist ein System aus einer ophthalmologischen Konsole und einer ophthalmologischen Kassette gemäß einem der Ansprüche 12 bis 13 vorgesehen.

In erfindungsgemäßer Weise ist zunächst erkannt worden, dass durch die Integration eines Strömungsleitelementes in die Drucksensoreinrichtung selbst, die Druckpulsationen verringert werden können und gleichzeitig die Druckpulsationen auf effektive Weise gemessen werden können. Ein weiterer Vorteil ist, dass das benötigte Bauraumvolumen gering ist.

Obwohl auf beliebige Drucksensoreinrichtung für ophthalmologische Kassetten anwendbar, wird die vorliegende Erfindung anhand von Drucksensoreinrichtungen für Phakoemulsifikationskassetten erläutert. Die Drucksensoreinrichtung kann insbesondere zylinderförmig ausgebildet sein.

Der Begriff "Strömungsleitmaßnahme" ist im weitesten Sinne zu verstehen und bezieht in der Beschreibung, vorzugsweise in den Ansprüchen, auf eine bauliche Maßnahme, die eine eingehende Strömung umleitet und/oder verändert.

Der Begriff "Strömungsleitelement" ist im weitesten Sinne zu verstehen und bezieht in der Beschreibung, vorzugsweise in den Ansprüchen, auf ein mechanisches Element, das innerhalb der Drucksensoranordnung angeordnet ist und eine eingehende Strömung entlang des Strömungsleitelements leiten kann.

Der Begriff Strömungsberuhigungselement ist im weitesten Sinne zu verstehen und bezieht in der Beschreibung, vorzugsweise in den Ansprüchen, auf ein mechanisches Element, das innerhalb der Drucksensoranordnung angeordnet ist und eine eingehende Strömung derart umlenkt, dass ein Druck der Strömung verringert wird. Insbesondere ist das Strömungsberuhigungselement ein statisches Element.

Die Drucksensormembran ist elastisch ausgebildet und wird durch ein einströmendes Fluid ja nach Druck des Fluids unterschiedlich stark ausgelenkt. Diese Auslenkung kann durch eine Messeinrichtung, die an der Konsole angeordnet ist, gemessen werden und ausgehend von der Auslenkung kann dann der Druck innerhalb der Drucksensoranordnung bestimmt werden.

Gemäß einer vorteilhaften Ausgestaltung der Erfindung ist der Drucksensoreinlass in der Wandung ausgebildet und der Drucksensorauslass in dem Boden ausgebildet. Somit wird eine Umlenkung der Strömung um annähernd 90° erreicht. Ein Vorteil hiervon ist, dass die Druckpulsationen weiter verringert werden können.

Es ist vorteilhaft, wenn die Strömungsleitmaßnahme ein Strömungsberuhigungselement, insbesondere eine Wand, ist. Die Wand kann beispielsweise als Prallmauer ausgebildet sein, gegen die die eingehende Strömung schlägt und anschließend um die Wand herum fließt. Auf diese Weise können eingehende Pulsationen des Drucks verringert werden. Ein weiterer Vorteil ist, dass eine eingehende Strömung in verschiedene Strömungspfade aufgeteilt werden kann, sodass sich kein kontinuierlicher Strömungspfad zwischen Drucksensoreinlass und Drucksensorauslass bildet.

Es ist ebenfalls denkbar, dass die Strömungsleitmaßnahme ein Strömungsleitelement, insbesondere ein von dem Drucksensoreinlass bis zu dem Boden reichendes Blech, ist. Die Strömungsleitmaßnahme kann ein Blech sein, das vor dem Drucksensoreinlass angeordnet ist und sich bis kurz vor und/oder bis zum Boden der Drucksensoreinrichtung erstreckt. Eine durch den Drucksensoreinlass eintretende Strömung, insbesondere eintretende Tropfen, wird durch das Strömungsleitelement bis zum Boden geleitet. Insbesondere kann das Strömungsleitelement die eintretende Strömung zum Drucksensorauslass leiten. Ein Vorteil hiervon ist, dass die Wahrscheinlichkeit verringert wird, dass die eintretende Strömung Luft aus der Drucksensoreinrichtung aufnimmt.

Gemäß einer bevorzugten Weiterbildung der Erfindung ist ein Abstand zwischen dem Drucksensoreinlass und der Strömungsleitmaßnahme geringer als 50%, vorzugsweise weniger als 30%, insbesondere weniger als 15%, des Abstandes zwischen dem Drucksensoreinlass und einer dem Drucksensoreinlass gegenüberliegenden Wandung. Mit anderen Worten ist die Strömungsleitmaßnahme nahe an dem Drucksensoreinlass angeordnet, insbesondere direkt vor dem Drucksensoreinlass. Ein Vorteil hiervon ist, dass eine stärkere Umlenkung der Strömung erreicht werden kann.

Vorteilhafterweise ist ein Innendurchmesser des Drucksensorauslasses geringer als 20%, vorzugsweise geringer als 10%, insbesondere geringer als 5% des Durchmessers der Drucksensoreinrichtung. Der Drucksensorauslass ist somit klein relativ zu dem Durchmesser der Drucksensoreinrichtung und entsprechend klein relativ zu dem Volumen der Drucksensoreinrichtung. Somit wirkt der Drucksensorauslass wie eine Drossel, die den statischen Druck der Strömung verringern kann und somit insbesondere Druckpulsationen reduzieren kann.

Gemäß einer vorteilhaften Ausgestaltung der Erfindung weist die Wandung einen äußeren Ring und einen inneren Ring mit eine Auflagefläche auf, wobei der innere

Ring eine geringere Höhe als der äußere Ring aufweist, wobei die Drucksensormembran auf der Auflagefläche angeordnet ist. Die Drucksensormembran ist somit auf der Oberseite der Drucksensoreinrichtung angeordnet. Insbesondere wird die Drucksensoreinrichtung fluidisch durch die Drucksensormembran abgedichtet. Ein Vorteil hiervon ist, dass die Drucksensormembran auf einfache Weise in die Kassette integriert werden kann.

Dabei ist es insbesondere von Vorteil, wenn die Oberseite der Drucksensormembran vollständig freiliegend ist. Die Oberfläche der Drucksensormembran ist insbesondere diejenige Seite der Drucksensormembran, die einem Innenraum der Drucksensoreinrichtung abgewandt ist. Mit anderen Worten ist die Drucksensormembran nicht von der Oberseite her eingeklemmt, sondern liegt lediglich auf der Wandung der Drucksensoreinrichtung auf. Beim Einsatz der Kassette in die Konsole kann dann eine entsprechende Messeinrichtung auf der Drucksensormembran angeordnet sein. Die Messeinrichtung kann insbesondere derart ausgebildet sein, dass sie die Drucksensormembran an den Randbereichen der Drucksensormembran einklemmt und diese so festlegt. Ein Vorteil hiervon ist, dass der Bauraum der Kassette verringert ist.

Es ist ebenfalls denkbar, dass eine Oberseite der Drucksensormembran zumindest teilweise durch einen Deckel der Drucksensoreinrichtung bedeckt ist. Der Deckel kann beispielsweise als Ringausgebildet sein, der die Randbereiche der Drucksensormembran einklemmt, hingegen den zentralen Mittelteil der Drucksensormembran freilässt, sodass die Messeinrichtung der Konsole daran angeordnet werden kann. Ein Vorteil hiervon ist, dass die die Kassette einfach hergestellt werden kann.

Gemäß einer bevorzugten Ausgestaltung der Erfindung ist die Drucksensormembran aus einem flexiblen Material hergestellt, wobei mittig in der Drucksensormembran eine Stahlscheibe angeordnet ist. Die Stahlscheibe kann das Element sein, an dem die Messeinrichtung der Konsole angeordnet werden kann. Ein Vorteil hiervon ist, dass die Messgenauigkeit der Drucksensoreinrichtung erhöht werden kann.

Des Weiteren ist es vorteilhaft, wenn eine Aspirationsleitung vorgesehen ist, die sich von einem Aspirationseinlass entlang des Anschlags und/oder entlang eines weiteren Anschlags zu einem Aspirationsauslass erstreckt, wobei an dem Aspirationsauslass ein weitere Drucksensoreinrichtung angeordnet ist. Mit anderen Worten kann die Aspirationsleitung eine analoge Drucksensoreinrichtung wie die Irrigationsleitung aufweisen. Insbesondere kann die Drucksensoreinrichtung der Aspirationsleitung gleich aufgebaut sein wie die Aspirationsleitung der Irrigationsleitung. Ein Vorteil hiervon ist, dass auch die Druckpulsationen der Aspirationsleitung verringert werden können. Ein weiterer Vorteil ist, dass der Augeninnendruck genauer kontrolliert werden kann.

Bevorzugt ist die Strömungsleitmaßnahme integral mit der Drucksensoreinrichtung verbunden. Beispielsweise kann die Strömungsleitmaßnahme mittels Spritzgusses zusammen mit dem Boden und der Wandung der Drucksensoreinrichtung hergestellt sein. Ein Vorteil hiervon ist, dass die Herstellungskosten verringert werden können.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: eine Drucksensoreinrichtung in Explosionsdarstellung gemäß einer Ausführungsform der vorliegenden Erfindung;
- Fig. 2: Die Drucksensoreinrichtung gemäß Figur 1 in einer Draufsicht;
- Fig. 3: Eine ophthalmologische Kassette gemäß einer Ausführungsform der vorliegenden Erfindung;
- Fig. 4: Ein System gemäß einer Ausführungsform der vorliegenden Erfindung.

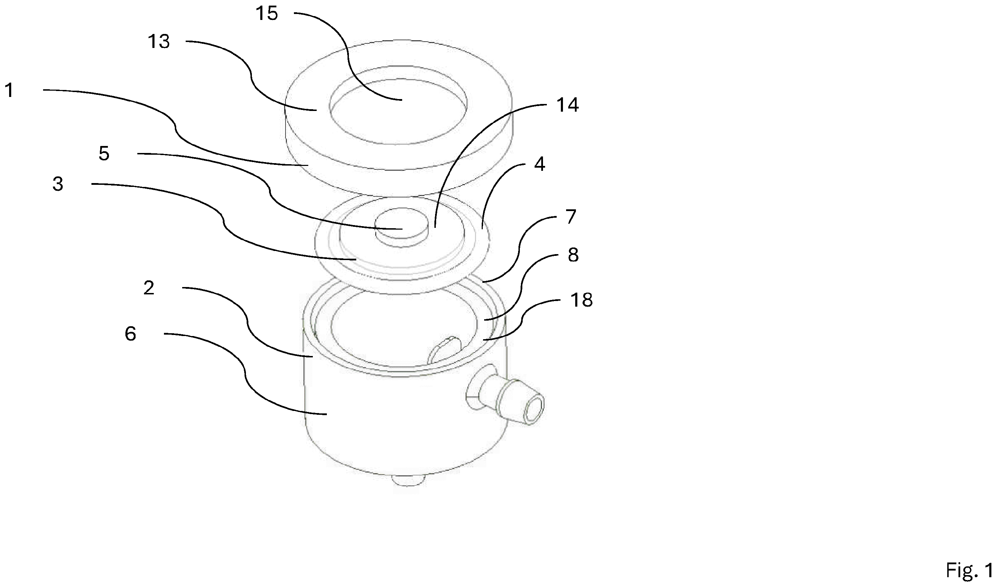

Fig. 1 zeigt eine Drucksensoreinrichtung in Explosionsdarstellung gemäß einer Ausführungsform der vorliegenden Erfindung.

Die Drucksensoreinrichtung 1 umfasst ein Drucksensorgehäuse 2 und eine Drucksensormembran 3. Die Drucksensormembran 3 ist scheibenförmig ausgebildet und hat eine scheibenförmige Grundplatte 4, die aus einem flexiblen Element hergestellt ist. Auf der Grundplatte ist eine scheibenförmige Erhöhung 14 vorgesehen, die ebenfalls aus einem flexiblen Element hergestellt ist. Mittig auf der scheibenförmigen Erhöhung 14 ist ein Stahlkern 5 angeordnet, dessen Höhe von einem Messinstrument erfassbar ist. Es ist ebenfalls denkbar, dass andere Messinstrumente vorgesehen sind, die den Druck mittels anderer physikalischer Größen ermitteln. Beispielsweise könnte ein Kraftsensor vorgesehen sein, der die auf die Drucksensormembran wirkende Kraft messen kann, sodass davon ausgehend der Druck bestimmbar ist.

Das Drucksensorgehäuse 2 ist zylindrisch ausgebildet mit einer kreisförmigen Wandung 6, die aus einem äußeren Ring 7 und einem inneren Ring 8 gebildet ist. Der äußere Ring 7 und der innere Ring 8 sind direkt aneinander angeordnet, wobei der innere Ring 8 eine geringere Höhe als der äußere Ring 7 aufweist. Insbesondere weist der innere Ring 8 einen Durchmesser auf, der zwischen 80% und 100%, vorzugsweise zwischen 90% und 100%, insbesondere zwischen 95% und 100% des Durchmessers der Drucksensormembran 3 entspricht. Somit kann die Drucksensormembran 3 auf dem inneren Ring 8, insbesondere einer Auflagefläche 18, angeordnet werden und wird dabei von dem äußeren Ring 7 positioniert.

Über der Drucksensormembran 4 ist ein Deckel 13 angeordnet. Dieser kann dazu genutzt werden, die Drucksensormembran in dem Drucksensorgehäuse 2 festzulegen. Der Deckel 13 ist ringförmig ausgebildet und kann über den äußeren Ring 7 gestülpt werden. Die zentrale Öffnung 15 des Deckels 13 ist größer als der Stahlkern 5, sodass ein Messgerät durch die zentrale Öffnung 15 und auf den Stahlkern 5 geführt werden kann.

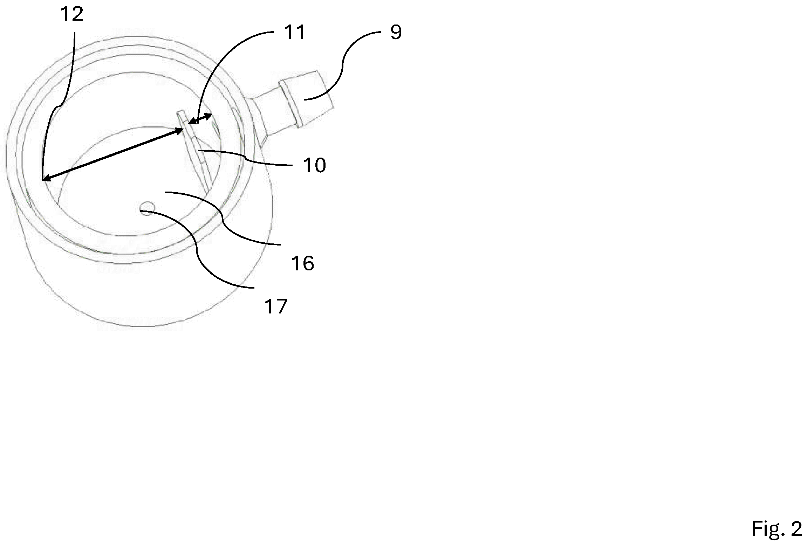

An der kreisförmigen Wandung 6 ist ein Drucksensoreinlass 9 angeordnet, durch den Fluide in das Drucksensorgehäuse 2 strömen können. An dem Drucksensoreinlass 9 ist ein Konnektor, hier in Form eines Luers 10 zur Verbindung mit einem Schlauchsystem vorgesehen. In dem Drucksensorgehäuse 9 ist eine Strömungsleitmaßnahme, hier in Form eines Strömungsberuhigungselements 10, angeordnet, hier in Form einer Wand, insbesondere einer Prallmauer. Die Prallmauer 10 ist in direkter Nähe zu dem Drucksensoreinlass 9 angeordnet, sodass ein einströmender Fluidstrahl durch die Prallmauer 10 umgelenkt wird. Direkte Nähe bedeutet dabei in Figur 1, dass ein Abstand 11 zwischen dem Drucksensoreinlass 9 und der Prallmauer 10 30% des Abstands 12 zwischen der Prallmauer 9 und der gegenüberliegenden Wandung beträgt. Dies ist in Figur 2 gezeigt. Die Prallmauer kann rechteckig sein und sich senkrecht zu einer Einflussrichtung des einströmenden Fluidstrahls erstrecken. Dabei kann die Prallmauer10 kontaktlos zu der Wandung 6 ausgebildet sein, das heißt, dass die Prallmauer 10 die Wandung 6 nicht kontaktiert.

Es ist ebenfalls denkbar, dass die Strömungsleitmaßnahme als eine Führung ausgebildet ist, die die eintretende Strömung bis zum Drucksensorauslass leitet. Im Gegensatz zu der Prallmauer, bei der die Strömung lediglich in beliebige, insbesondere undefinierte und/oder zufällige, Richtungen umgelenkt wird, wird bei der Führung die Strömung entlang ihrer Länge geführt. Am Boden der Drucksensoreinrichtung kann sich Flüssigkeit befinden, in die die Strömung geleitet wird. Somit entstehen keine Tropfen beziehungsweise Spritzer von Flüssigkeit innerhalb der Drucksensoreinrichtung, die Luft aus der Drucksensoreinrichtung aufnehmen könnten.

Figur 2 zeigt die Drucksensoreinrichtung gemäß Figur 1 in einer Draufsicht.

In einem Boden 16 der Drucksensoreinrichtung 1 ist ein Drucksensorauslass 17 ausgebildet. Der Drucksensorauslass 17 ist mittig des Bodens 16 angeordnet und kann analog zu dem Drucksensoreinlass 9 einen Konnektor in Form eines Luers (nicht gezeigt) umfassen.

Die Drucksensoreinrichtung 1 ist insbesondere dazu geeignet, in einem ophthalmologischen System mit einer Rollenpumpe eingesetzt zu werden. Durch die Rollenpumpe wird ein pulsierender Fluidstrom bereitgestellt, dessen Druck gemessen werden soll. Dabei sollen die Pulsationen des Fluidstroms minimiert werden. Hierfür wird die Drucksensoreinrichtung 1 genutzt. Der Fluidstrom kann durch den Drucksensoreinlass 9 in das Drucksensorgehäuse 2 strömen. Durch die Prallmauer wird der Fluidstrom umgelenkt und durch das im Vergleich zum eintretenden Fluidstrom große Innenvolumen des Drucksensorgehäuses gedrosselt. Hierdurch werden die Druckpulsationen des eintretenden Fluidstroms verringert.

Der eintretende Fluidstrom sammelt sich in dem Drucksensorgehäuse und drückt die Drucksensormembran 3 nach außen, in Figur 1 also nach oben. Diese Auslenkung ist abhängig von dem Druck innerhalb des Drucksensorgehäuses. Durch einen Messstab kann diese Auslenkung gemessen werden und somit der Druck in dem Drucksensorgehäuse bestimmt werden.

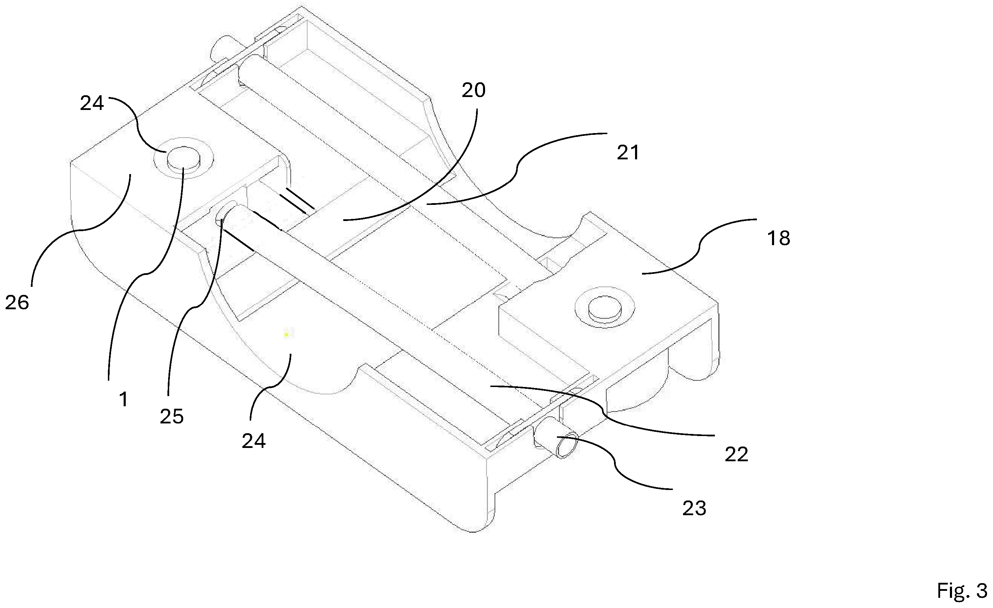

Figur 3 zeigt eine ophthalmologische Kassette gemäß einer Ausführungsform der vorliegenden Erfindung.

Die Kassette 20 umfasst eine Aspirationsleitung 21 und eine Irrigationsleitung 22. Da die Aspirationsleitung 21 und die Irrigationsleitung 22 gleich ausgebildet sind und lediglich spiegelverkehrt aneinander angeordnet sind, wird Figur 1 lediglich in Bezug auf die Irrigationsleitung 22 beschrieben.

Die Irrigationsleitung 22 umfasst einen Einlass 23 und verläuft anschließend entlang eines Anschlags 24, auf den eine Rollenpumpe aufsetzbar ist. Durch das Drehen der Rollenpumpe wird die Irrigationsleitung 22 gewalkt, sodass ein Fluid in der Irrigationsleitung gefördert wird. Die Irrigationsleitung verläuft anschließend bis zu einem Auslass 25, der in einer Drucksensoreinrichtung 1 mündet. Im Gegensatz zu der Drucksensoreinrichtung 1 gemäß Figur 1 umfasst die Drucksensoreinrichtung 1 gemäß Figur 3 keinen Deckel. Stattdessen wird die Drucksensormembran 3 durch das Gehäuse 26 der Kassette 20 eingeklemmt.

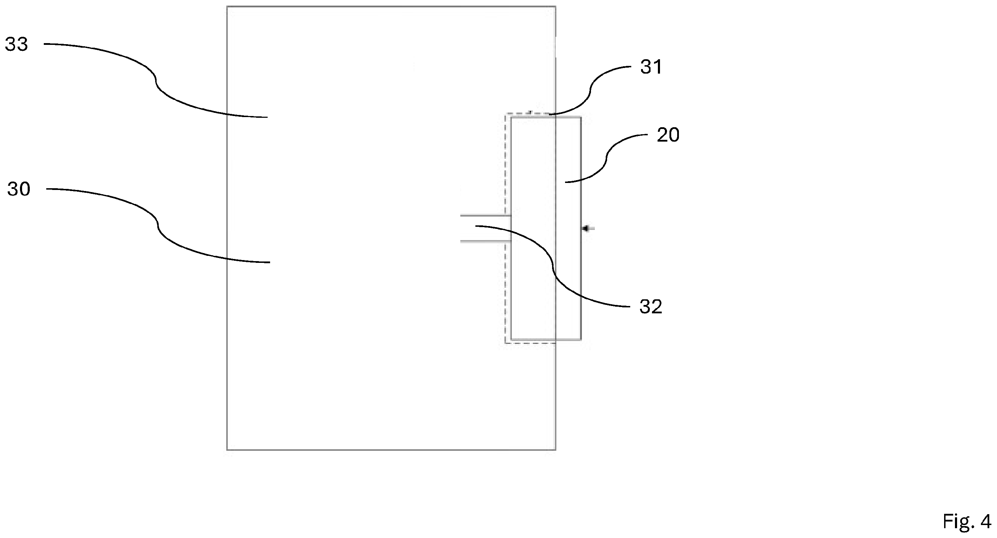

Figur 4 zeigt ein System gemäß einer Ausführungsform der vorliegenden Erfindung.

Das System 33 umfasst eine ophthalmologische Konsole 30 mit einem Schacht 31, in dem Schacht 31 ist eine Kassette 20 angeordnet. In der Konsole 30 ist eine Messeinrichtung 32 angeordnet, die dazu ausgebildet ist, eine Auslenkung des Stahlkerns der Drucksensoreinrichtung (nicht gezeigt) der Kassette zu erfassen. Ausgehend von der Auslenkung kann der Druck innerhalb der Kassette, insbesondere in der Aspirationsleitung und/oder der Irrigationsleitung gemessen werden.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Drucksensoreinrichtung
- 2: Drucksensorgehäuse
- 3: Drucksensormembran
- 4: Grundplatte
- 5: Stahlkern
- 6: Wandung
- 7: Äußerer Ring
- 8: Innerer Ring
- 9: Drucksensoreinlass
- 10: Prallmauer
- 11: Abstand
- 12: Abstand
- 13: Deckel
- 14: Erhöhung
- 15: Zentrale Öffnung
- 16: Boden
- 17: Drucksensorauslass
- 18: Auflagefläche
- 20: Kassette
- 21: Aspirationsleitung
- 22: Irrigationsleitung
- 23: Einlass
- 24: Anschlag
- 25: Auslass
- 26: Gehäuse

- 30: Konsole
- 31: Schacht
- 32: Messeinrichtung
- 33: System

## Patentansprüche

1. Drucksensoreinrichtung (1) für eine ophthalmologische Kassette, mit einem Drucksensoreinlass (9) und einem Drucksensorauslass (17), wobei die Drucksensoreinrichtung (1) zumindest einen Boden (16) und eine Wandung (6) aufweist,
**dadurch gekennzeichnet, dass** die Drucksensoreinrichtung (1) mit einer Drucksensormembran (3) ausgebildet ist, wobei in der Drucksensoreinrichtung (1) eine Strömungsleitmaßnahme (10) angeordnet ist.

2. Drucksensoreinrichtung (1) gemäß Anspruch 1, **dadurch gekennzeichnet, dass** der Drucksensoreinlass (9) in der Wandung (6) ausgebildet ist und dass der Drucksensorauslass (17) in dem Boden (16) ausgebildet ist.

3. Drucksensoreinrichtung (1) gemäß Anspruch 1 oder 2, **dadurch gekennzeichnet, dass** die Strömungsleitmaßnahme (10) ein Strömungsberuhigungselement, insbesondere eine Wand, ist.

4. Drucksensoreinrichtung (1) gemäß einem der Ansprüche 1 bis 3, **dadurch gekennzeichnet, dass** die Strömungsleitmaßnahme (10) ein Strömungsleitelement, insbesondere ein von dem Drucksensoreinlass bis zu dem Boden (16) reichendes Blech, ist.

5. Drucksensoreinrichtung (1) gemäß einem der Ansprüche 1 bis 4, **dadurch gekennzeichnet, dass** ein Abstand (11) zwischen dem Drucksensoreinlass (9) und der Strömungsleitmaßnahme(10) geringer ist als 50%, vorzugsweise weniger als 30%, insbesondere weniger als 15%, des Abstandes (12) zwischen dem Drucksensoreinlass (9) und einer dem Drucksensoreinlass (9) gegenüberliegenden Wandung.

6. Drucksensoreinrichtung (1) gemäß einem der Ansprüche 1 bis 5, **dadurch gekennzeichnet, dass** ein Innendurchmesser des Drucksensorauslasses (17) geringer ist als 20%, vorzugsweise geringer als 10%, insbesondere geringer als 5% des Durchmessers der Drucksensoreinrichtung (1).

7. Drucksensoreinrichtung (1) gemäß einem der Ansprüche 1 bis 6, **dadurch gekennzeichnet, dass** die Wandung (6) einen äußeren Ring (7) und einen inneren Ring (8) mit eine Auflagefläche aufweist, wobei der innere Ring (7) eine geringere Höhe als der äußere Ring (8) aufweist, wobei die Drucksensormembran (3) auf der Auflagefläche angeordnet ist.

8. Drucksensoreinrichtung (1) gemäß Anspruch 7, **dadurch gekennzeichnet, dass** eine Oberseite der Drucksensormembran (3) vollständig freiliegend ist.

9. Drucksensoreinrichtung (1) gemäß Anspruch 7, **dadurch gekennzeichnet, dass** eine Oberseite der Drucksensormembran (3) zumindest teilweise durch einen Deckel (13) der Drucksensoreinrichtung (1) bedeckt ist.

10. Drucksensoreinrichtung (1) gemäß einem der Ansprüche 1 bis 9, **dadurch gekennzeichnet, dass** die Drucksensormembran (3) aus einem flexiblen Material hergestellt ist, wobei mittig in der Drucksensormembran (3) eine Stahlscheibe (5) angeordnet ist.

11. Drucksensoreinrichtung (1) gemäß einem der Ansprüche 1 bis 10, **dadurch gekennzeichnet, dass** die Strömungsleitmaßnahme(10) integral mit der Drucksensoreinrichtung (1) verbunden ist.

12. Ophthalmologische Kassette (20), insbesondere für ein Phakoemulsifikationssystem, umfassend ein Gehäuse (26), eine Irrigationsleitung (22) und einen Anschlag (24) für eine Rollenpumpe, wobei sich die Irrigationsleitung (22) von einem Einlass (23) entlang des Anschlags (24) bis zu einem Auslass (25) erstreckt, wobei in dem Auslass (25) eine Drucksensoreinrichtung (1) gemäß einem der Ansprüche 1 bis 11 angeordnet ist.

13. Ophthalmologische Kassette (20) gemäß Anspruch 12, **dadurch gekennzeichnet, dass** eine Aspirationsleitung (21) vorgesehen ist, die sich von einem Aspirationseinlass entlang des Anschlags und/oder entlang eines weiteren Anschlags zu einem Aspirationsauslass erstreckt, wobei an dem Aspirationsauslass ein weitere Drucksensoreinrichtung (1) angeordnet ist.

14. System aus einer ophthalmologischen Konsole und einer ophthalmologischen Kassette (20) gemäß einem der Ansprüche 12 bis 13.
